# EUROPEAN PATENT SPECIFICATION

(11) **EP 1 076 240 B1**
(45) Date of publication and mention of the grant of the patent: **14.02.2007**
(21) Application number: 00906661.4
(22) Date of filing: 03.03.2000
(51) Int. Cl.: G01N 33/53, G01N 33/543

(54) **METHOD FOR ASSAYING CRP WITH THE USE OF PHOSPHORYLCHOLINE**
VERFAHREN ZUM BESTIMMEN VON CRP MIT HILFE VON PHOSPHORYLCHOLIN
METHODE DE DOSAGE DE CRP A L'AIDE DE PHOSPHORYLCHOLINE

(30) Priority: 03.03.1999 JP 5535299
(43) Date of publication of application: 14.02.2001
(73) Proprietor: ORIENTAL YEAST CO., LTD., Tokyo 174-8505 (JP); Tamura, Hiroshi, Tokorozawa-shi, Saitama 359-0004 (JP); Lee, Y., C., Timonium, MD 21093 (US)
(72) Inventor: TAMURA, Hiroshi, Tokorozawa-shi, Saitama 359-0004 (JP); LEE, Y., C., Timonium, MD 21093 (US); KAWAGUCHI, Kichitaro, DECEASED (JP); TAKAGAHARA, Isamu, Tsukuba-shi, Ibaraki 300-2635 (JP); MATUO, Yuhsi, Suita-shi, Osaka 565-0851 (JP); SAITO, Keiko, Baltimore, MD 21215 (US)
(74) Representative: HOFFMANN EITLE
(86) International application number: PCT/JP2000/001268
(87) International publication number: WO 2000/052469

(56) References cited:
- GB-A- 2 217 335
- JP-A- 9 241 233
- JP-A- 62 192 662
- J. LIBERDA ET AL.: "Application of biotinylated phosphorylcholine polyacrylamide derivative in the study of binding properties of boar seminal plasma proteins" CHEM. PAPERS, vol. 52, 1998, pages 323-324, XP002201848
- K. SAITO ET AL.: "Quantification of Eu3+ in Quantum-Dye-Labeled Materials by Ashing and Dissociation Enhancement" ANAL. BIOCHEM., vol. 258, - 1998 pages 311-314, XP002201850
- PATENT ABSTRACTS OF JAPAN vol. 012, no. 138 (P-695), 27 April 1988 (1988-04-27) & JP 62 259063 A (ORIENTAL YEAST CO LTD;OTHERS: 01), 11 November 1987 (1987-11-11)
- ROBEY F A ET AL: "SYNTHESIS AND USE OF NEW SPIN LABELED DERIVATIVES OF PHOSPHORYL CHOLINE IN A COMPARATIVE STUDY OF HUMAN DOGFISH MUSTELUS-CANIS AND LIMULUS-POLYPHEMUS C REACTIVE PROTEINS" JOURNAL OF BIOLOGICAL CHEMISTRY, vol. 258, no. 6, 1983, pages 3895-3900, XP001080190 ISSN: 0021-9258
- J. LIBERDA ET AL.: "Preparation of Biotinylated and FITC-Labelled Phosphorylcholine Poly(acrylamide) Derivatives and Their Application for Protein Ligand-Binding Studies" CHIMIA, [Online] vol. 53, 1999, pages 528-532, XP001080195 Retrieved from the Internet: <URL:www.chimia.ch/issues/toc/9911/toc9911 .html> [retrieved on 2002-06-05]
- SHIVA BERMAN ET AL.: 'Binding of C-reactive protein to nucleated cells leads to complement activation without cytolysis' THE JOURNAL OF IMMUNOLOGY vol. 136, no. 4, 15 February 1986, CHICAGO, pages 1354 - 1359, XP002929676

## Description

### TECHNICAL FIELD

The present invention relates to a novel method for determining levels of C-reactive protein (referred to as "CRP" hereinafter). More specifically, the invention relates to a novel method for effecting micro-quantitative analysis of CRP, using labeled phosphorylcholine (referred to as "PC" hereinafter).

### BACKGROUND OF THE INVENTION

CRP is a protein which is reactive with C-polysaccharide in the capsule of Diplococcus pneumoniae, and is present in the β-globulin fraction in serum. It consists of 5 identical peptides, each of which has a molecular weight of about 130 kDa. The concentration of CRP in the blood of a healthy human is below about 350 ng/ml, and dramatically increases to as much as 0.1 mg/ml during acute phase reactions such as occur during infection, inflammation or with tissue damage. CRP is thus recognized as an acute phase protein [Agrawal, A., Kilpartrick, J. M., and Volanakis, J. E. (1994) in Acute Phase Proteins-Molecular Biology, Biochemistry and Clinical Applications-(Mackiewicz, A., Kushuner, I., and Baumann, H., eds.), pp. 79-92, CRC Press, London]. As a result of its characteristics, as mentioned above, CRP has been conventionally used as a diagnostic marker indicating bacterial infection, inflammation and for post-surgery prognosis.

Although the biological role of CRP has not yet been completely elucidated, it has gradually been ascertained that CRP exerts various pro-inflammatory host-protection activities including complement activation [Richards, R. L., et al., (1997) Proc. Natl. Acad. Sci. USA 74, 5672-5676; Richards, R. L., et al., (1979) J. Immunol. 122, 1185-1189; Miyazawa, K., and Inoue, K. (1990) J. Immunol. 145, 650-654; Jiang, H., et al., (1991) J. Immunol. 146, 2324-2330; Wolbink, G. J., et al., (1996) J. Immunol. 157, 473-479] and lymphocyte opsonization [Mortensen, R. F., et al., (1976) J. Immunol. 117, 774; Edwards, W. M., et al., (1982) J. Immunol. 128, 2498; Szalai, A. J., et al., (1995) J. Immunol. 155, 2557-2563]. It is likely that these activities make contributions to non-specific host protection in the pre-immune phase of an inflammatory reaction.

In order to diagnose bacterial infection, inflammation and to assist in post-surgery prognosis, it is necessary for an acute phase protein level to be assayed accurately not only in an acute phase but also for it to be assayed accurately when a person is in a healthy state. CRP is an acute phase protein most sensitive and most highly reactive to extraneous stimuli [Morley, J. J., and Kushner, L. (1982) Ann. N. Y. Acad. Sci. 389, 406-418]. Therefore, the ability to accurately assay CRP levels in a healthy individual, will enable the early stages of infection to be diagnosed more accurately. The early increase of CRP level at acute phase essentially needs to be detected in a rapid and accurate manner in order to monitor therapeutic treatment and to diagnose prognosis, wherein the detection requires a highly sensitive method capable of assaying serum CRP level within the normal range.

It is known that using a nephelometric immunoassay method [Yamamoto, S., et al., (1994) Vet. Quarterly 16, 74-77; Yamamoto, S., et al., (1993) Vet. Immunol. Immunopathol. 36, 257-264] or latex agglutination test [Sarikaputi, M., et al., (1992) Jap. J. Vet. Res. 40, 1-12; Yamada, T., et al., (1993) Ann. Clin. Biochem. 30, 72-76] CRP levels can be determined within a range of 1 to 1000 µg CRP/ml and within a range of 10 to 400 µg CRP/ml, respectively, in a simple manner. These ranges include the typical serum range (10 to 100 µg CRP/ml) in an acute phase. Since blood CRP concentration in a healthy individual is below about 350 ng/ml as stated above, however, the aforementioned methods are incapable of assaying CRP concentrations within a normal range. Although these methods have been used widely at clinical tests, they are not sufficiently sensitive to detect early CRP level elevation at the beginning of an acute phase. Thus, assay results are simply expressed as "negative", "positive" or "elevation". In addition to the assay of CRP level at peak in acute phase, precise assay of normal CRP level or CRP level at the onset of acute phase is extremely useful so as to enhance the precision of diagnosis.

Compared with the aforementioned methods, fluorescent immunoassay (FIA) [Siboo, R., and Kulisek, E. A. (1976) J. Immunol. Methods 23, 59-67] and radioimmunoassay [Claus, D. R., et al., (1976) J. Lab. Clin. Med. 87, 120-128] are considerably more sensitive. However, they are not sufficiently sensitive to effect assay of serum CPR in a healthy individual. Enzyme-linked immunosorbent assay (ELISA) [Lamb, W. R., et al., (1991) Med. Lab. Sci. 48, 201-205; Shields, M., et al., (1991) J. Immunol. Methods 141, 253-261; Yamamoto, S., et al., (1994) Vet. Quarterly 16, 74/77] is based on the same principle as those employed in both FIA and RIA, and represents a development of these methods. ELISA is a quantitative assay method, generally having greater sensitivity than that of RIA. When CRP concentration is assayed, however, no significant difference is found between the CRP range assayed by ELISA and the range assayed by RIA. Specifically, compared with the range of 8 to 800 ng CRP/ml as detected by RIA [Claus, D. R., et al., (1976) J. Lab. Clin. Med. 87, 120-128], the range detected by ELISA is 10 to 100 ng CRP/ml [Unten, S. K., and Hokama, Y. (1987) J. Clin. Lab. Anal. 1, 136-139]. These results are indicative of the fact that ELISA for CRP assay needs to be greatly modified.

Liberda, J. et al (1998) Chem. Papers 52:323-324 discloses determination of CRP using biotin-labeled PC.

Robey, F.A. et al (1983) J. Biol. Chem. 258:3895-3900 describes the use of spin labeled derivatives of PC in assays for CRP.

GB 2217225 describes detection of CRP in test samples employing a system where PC residues are first immobilized on a solid phase, CRP is then bound to the immobilized PC and finally a second CRP-binding signal substance is bound for detection.

### SUMMARY OF THE INVENTION

The purpose of the present invention is to provide a method for rapidly determining CRP in a sample which includes not only a CRP concentration in the serum occurring in an acute phase, but also serum CRP which is within the normal range, with a high degree of sensitivity.

Another purpose of the present invention is to develop a method for rapidly assaying CRP in a sample with a high degree of sensitivity and which receives no influence from frompentraxin family proteins having high homology to CRP present in serum, using phosphorylcholine (PC), and wherein the method is applicable in clinical diagnosis.

### BRIEF DESCRIPTION OF THE DRAWINGS

Fig. 1 shows a method for determining CRP using Eu³⁺-labeled probe, wherein (A), (B) and (C) show the results of the assay of CRP concentration by an antibody-antibody method, antibody-PC method and PC-antibody method, respectively;
Fig. 2 shows standard curves for CRP assay, as analyzed by using the antibody-antibody method (A), the antibody-PC method (B) and the PC-antibody method (C);
Fig. 3 shows the results of CRP assay in 100-fold diluted human serum obtained by using each of the above methods;
Fig. 4 shows the results of CRP assay in 100-fold diluted serum by the antibody-antibody method (A) and the antibody-PC method (B);
Fig. 5 shows the quantitative precipitation of CRP with PC₅₈-BSA;
Fig. 6 shows the comparison in CRP-binding activity between PC₅₈- and PC_{γ}-BSA ;
Fig. 7 shows the inhibition of the PC-binding activity of CRP by water-soluble PC; and
Fig. 8 shows specific and non-specific CRP bindings according to the antibody-PC method (B).

### DETAILED DESCRIPTION OF THE INVENTION

The present inventors have studied extensively various CRP assay methods. As a result of such studies, the inventors have found that proteins of the pentraxin family, such as amyloid A and amyloid P, are present in human serum and these proteins react with anti-CRP antibodies. It is suggested that one of the factors inhibiting the elevation of the assay sensitivity for the assay of CRP concentration by ELISA is the reactivity of the proteins with anti-CRP antibodies and that it is therefore very difficult to accurately determine CRP in, for example, human serum samples.

Focussing on the reactivity of CRP with C-polysaccharide existing in the capsule of Diplococcus pneumonia to bind to the phosphorylcholine group of the C-polysaccharide, the present inventors have determined the possibility of realizing highly sensitive assay of CRP concentration, by utilizing the specific binding of CRP to phosphorylcholine.

In accordance with the present invention, an assay is thus performed by a sandwich assay method using the specific binding of CRP to phosphorylcholine (PC). In a preferred embodiment, PC is labeled and then used. In such a case, PC may be directly labeled or indirectly labeled by conjugating another molecule to PC, wherein said molecule mediates a labeling means. Additionally, PC may be labeled with a radioactive labeling means or with a non-radioactive labeling means. Further, these labeling means are not limited to currently available labeling means.

As a radioactive labeling means for labeling PC, conventional radioisotopes such as ¹²⁵I and ³²P ca be used. As a non-radioactive labeling means for labeling PC, any of the following methods can be used: methods using enzymes, such as alkali phosphatase (AP), horse radish peroxidase, β-galactosidase and glucose oxidase; methods using luminescence from luciferin with luciferase; and methods using fluorescent dyes such as fluorescein and lanthanide. However, it should be noted that since the use of radioactive materials may not be preferable in conducting examination on site in clinical practice, the use of a non-radioactive labeling means is generally preferable. In one embodiment of the invention, lanthanum-series elements (lanthanides) are particularly used as fluorescent substances.

Lanthanides have been drawing attention in recent years as fluorescent probes for use in assaying biologically active compounds (for example, enzyme, lectin, receptor, and the like) and for histochemical research studies. Lanthanides emitting fluorescence, samarium (Sm³⁺), europium (Eu³⁺), and terbium (Tb³⁺) are known [Saito, K., et al., (1998) Anal. Biochem., 258, 311-314; Elliot, C. T., et al., (1994) Analyst, 119, 2565-2569; Pettersson, K., et al., (1993) Clin. Chem., 39, 2084-2089; Rothlin, M. A., et al., (1993) Cancer, 71, 701-707; Samiotaki, M., et al., (1997) Anal. Biochem., 253, 156-161]. One of the advantages of utilizing fluorescence emitted from such lanthanides is that sensitivity at the same level as or at a level higher than that recovered by the methods utilizing radioisotopes can be obtained.

Any lanthanide which emits fluorescence can be utilized as fluorescent substance in labeling PC with lanthanides in the present invention. Among them, the use of Eu³⁺ is preferable. Eu³⁺ exhibits high sensitivity, has a long fluorescence life (far longer than the mean background fluorescence by FIA), and, in addition, the shelf life of Eu³⁺ labeling reagents is far longer than that of RIA reagents.

The present invention will now be described herein below with reference to an example of the assay method of CRP concentration by a dissociation-enhanced lanthanide fluorescent immunoassay (DELFIA) method [Guzaeva, T. V.., et al., (1993) Immunology Lett. 35, 285-290] using lanthanides, particularly Eu³⁺.

In order to label PC with Eu³⁺, the method described in the report of Saito, K., et al. (Saito, K., et al., (1998) Anal. Biochem. 258, 311-314) is used. According to this method, in order to label protein or amino acid with Eu³⁺, various chelating substances can be used, for example an EDTA phenylisothiocyanate derivative N¹-(p-isothiocyanatebenzyl)-diethylenetriamine-N¹, N², N³, N³-tetraacetic acid (DETATA-ITC) represented by the following formula: 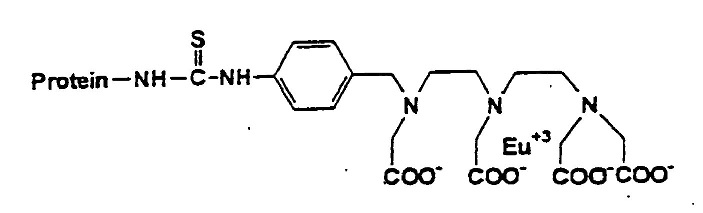 or diethylenetriamine-N¹, N², N³-pentaacetic acid (DTPA) represented by the following formula: 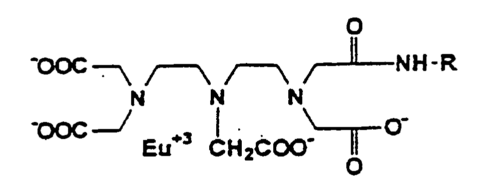 [in the formula, R represents aspargine-bound type sugar chain as represented by the following formula: 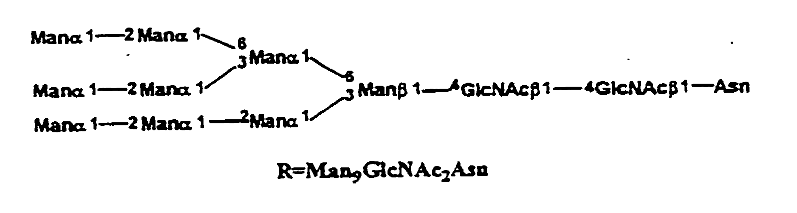 ] or dianhydride thereof (DTPAda). The protein can be labeled with Eu³⁺ by binding these chelating substances by way of the functional groups thereof to a protein and the like.

In order to label PC ([(CH₃)₃NCH₂CH₂OPO₃H₂]⁺) with these chelating substances in accordance with the present invention, a protein having no influence on the reaction is first bound to PC to prepare a PC-protein complex. Once this is achieved, the chelating substances are bound to the protein. A protein may also be first labeled and then bound to PC. The protein is bound to PC by substituting the amino group of the protein with the PC group. Any compound which lacks an amino group can also be used provided that the compound can introduce substance chelating lanthanides.

It is required that the protein to be bound to PC is able to bind a single or multiple molecules of Eu^{3+,} and must have no influence on the reaction. Any protein which satisfies these conditions can be used, and they include, but are not limited to, any available protein, for example bovine serum albumin (BSA), keyhole limpet hemocyanine (KLH), ovalbumin (OVA) and rabbit serum albumin. More preferably, BSA or KLH are used. Most preferably, BSA is used. A person having ordinary skill in the art will readily understand that substances other than proteins are also applicable for the purpose under discussion.

Using chelating substances, PC was labeled with Eu³⁺ by using DTPAda in accordance with the method reported above, namely, that of Kawasaki, N., and Lee, Y. C. (1997) Anal. Biochem. 250, 260-262. PC₃₅-BSA and PC₅₈-BSA, and BSA respectively contain 7-, 6- and 4 molecules of Eu³⁺ per one molecule of the protein. PC₃₅-BSA as used herein means a conjugate where 35 PC molecules on average are bound per one BSA molecule; PC₅₈-BSA herein means a conjugate where 58 PC molecules on average are bound per one BSA molecule. The quantity of the bound PC is almost equal to the quantity of the Eu³⁺ bound to IgG. When protein, for example anti-hCRP polyclonal rabbit IgG (molecular weight of 160 kDa) is labeled by a conventional method, 9 atoms of Eu³⁺ are bound per one molecule of the protein.

In accordance with the method of the present invention, Eu³⁺ for fluorometry is first dissociated from an initial chelating substance to form a complex, along with 1-(β-naphthyl)-3,3,3-trilfluoroacetone (β-NTA). The complex emits intense fluorescence in the presence of a surfactant (the fluorescence is referred to simply as Eu³⁺ fluorescence hereinafter). The duration of the attenuation of Eu³⁺ fluorescence is longer by 10⁴-fold, as compared with polystyrene and other plastics and glass materials. Thus, the time-resolved fluorescence of Eu³⁺ in a microtiter plate can be detected with a time-resolved fluorescence microtiter plate reader (Victor, Model 1420, Wallac Co., Gaithersburg, MD) (time-resolved fluorometry). Because Eu³⁺ fluorescence can be detected following irradiation with an excitation beam for a given period of time with subsequent sufficient attenuation being conducted for the fluorescence emitted from the immobilizing phase, any adverse influence of background fluorescence can be eliminated.

Specific conditions for detecting fluorescence (in cps) from Eu³⁺ dissociated from the chelating substance bound to the protein may be determined appropriately. As one example, Eu³⁺ fluorescence is detected by means of single photon counting, using a xenon flash lamp (1,000 Hz) for a total counting time of 1 second, with a 50-µs delay time and a 250-µs counting time per flash (λex = 340 nm; λem = 615 nm), after 15-min incubation in a 200 µl/well enhancement solution [Hemmila, I., et al., (1984) Anal. Biochem. 137, 335-343] at ambient temperature. For CRP assay and PC-binding assay by the time-resolved fluorescence immunoassay (TR-FIA) method, the value of the CRP concentration in 20 mM EDTA-TBST was used as a background value.

When Eu³⁺ dissociation hardly occurs due to the intense binding of Eu³⁺ to the chelating substance, the labeled sample may be recovered from the plate, and then the sample may be burned, to modify all the organic materials therein into ashes containing only inorganic salts, to thereby enable assay of the Eu³⁺ concentration to be effected. In determining Eu³⁺ concentration by means of ash preparation, a method essentially established for determining organic phosphoric acid is used [Ames, B. N. and Dubin, D. T., (1960) J. Biol. Chem., 235, 769-758]. The ash sample is again dissolved by means of incubation in 1.5 ml of 0.1 M acetic acid at 55°C for 30 minutes. The Eu³⁺ fluorescence in an appropriate volume of the dissolved sample (corresponding to 20 to 200 fmol Eu³⁺) is then able to be detected by the DELFIA method. Based on the results, the Eu³⁺ concentration can be assayed accurately even after ash preparation [Saito, K., et al., (1998) Anal. Biochem. 258, 311-314].

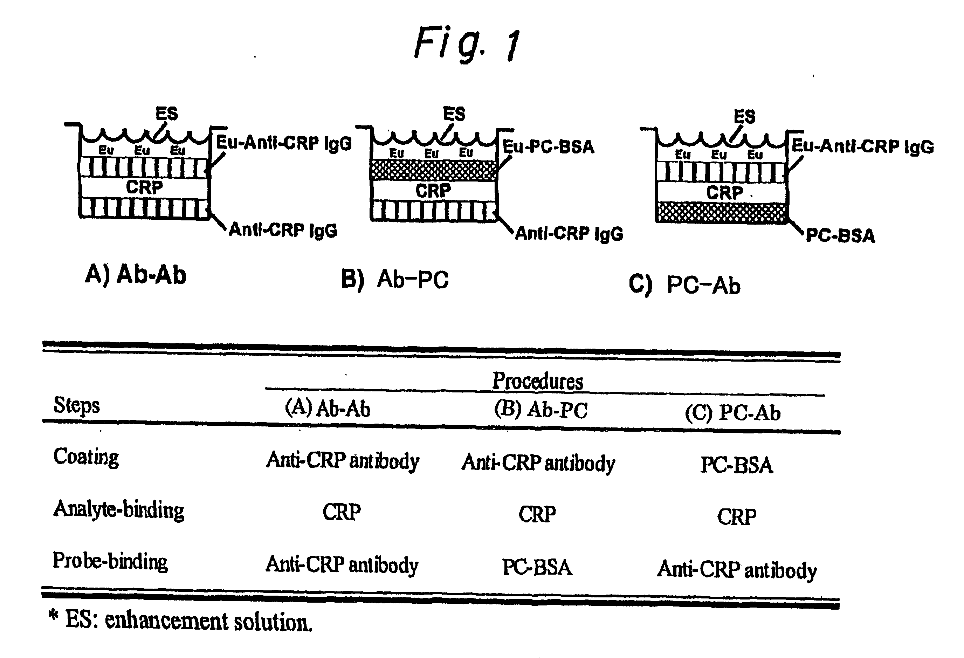

Sandwich assay methods for determining CRP concentration in samples by using various labeling means are broadly grouped into an antibody-antibody method (A) as a sandwich assay using two species of antibodies and sandwich assay methods [(B) and (C)] using PC (see Fig. 1).

The method for determining CRP concentration by the antibody-antibody method (A) comprises the steps of:
i) immobilizing an anti-CRP antibody on a plastic plate;
ii) reacting a sample solution in which the CRP concentration is intended to be assayed, with the anti-CRP antibody; and
iii) reacting a labeled anti-CRP antibody with the sample solution to thereby determine the CRP concentration in the sample [Fig. 1 (A)].

The inventors have nevertheless found that CRP present in samples obtained from humans, particularly serum CRP can not be accurately assayed by CRP assay using the antibody-antibody method (A). The reason is probably that in human serum amyloid A (hSAA) and P(hSAP) may react with anti-CRP antibodies. More specifically, hSAA and hSAP are fairly homologous to human CRP (66% and 70%, respectively) [Agrawal, A., et al., (1994) in Acute Phase Proteins-Molecular Biology, Biochemistry and Clinical Applications-(Mackiewicz, A., et al., eds.), pp. 79-92,. CRC Press, London; Shrive, A. K., et al., (1996) Nature Struc. Biol. 3, 346-354; Kilpatrick, J., and Volanakis, J. (1991) Immunol. Res. 10, 43-53]. Accordingly, hSAA and hSAP may bind to the anti-CRP antibodies to be used for the assay. When used, anti-CRP polyclonal antibodies particularly involve difficulty in the elimination of the influence of the pentraxins contained in serum.

In accordance with the present invention, therefore, a sandwich assay method using only one anti-CRP antibody species and PC in place of the other antibody species is employed, so as to provide an assay method which is not subject to any of the aforementioned drawbacks. Two types of sandwich assay methods using PC are described herein, namely antibody-PC method (B) and PC-antibody method (C). The antibody-PC method (B) comprises the steps of:
i) immobilizing an anti-CRP antibody on a plastic plate;
ii) reacting a sample solution in which the CRP concentration is intended to be assayed, with the anti-CRP antibody; and
iii) reacting a labeled PC with the sample solution to determine the quantity of the label bound to the immobilizing phase to determine the CRP concentration in the sample [Fig. 1 (B)]. Alternatively, the PC-antibody method (C) comprises the steps of:

i) immobilizing PC on an immobilizing phase such as a plastic plate;
ii) reacting a sample solution in which the CRP concentration is intended to be assayed with PC; and
iii) reacting an anti-CRP antibody with the sample solution to assay the quantity of the label bound to the immobilizing phase and thereby to determine the CRP concentration in the sample [Fig. 1 (C)].

In accordance with the present invention, reaction between CRP and PC is performed at least once by such a sandwich method. Because PC is not reactive with other pentraxin family members, unlike anti-CRP antibodies, the influence of undesirable reactions of PC with hSAA and hSAP, which is disadvantageous for the antibody-antibody method, can be prevented.

In accordance with the present invention, so as to enhance assay sensitivity, the sandwich assay antibody-PC method (B) is used. The PC-antibody method (C) has a drawback in that it causes a reduction in assay sensitivity, because serum lecithin blocks the PC-binding site of CRP in the presence of calcium at the reaction stage between the PC on the immobilizing phase and the CRP in a serum sample. While, according to the antibody-PC method (B), the inhibition of the reaction due to serum lecithin and calcium can be prevented because PC is able to interact with CRP after washing the immobilizing phase after the CRP in a serum sample is bound to the anti-CRP antibodies bound to the immobilizing phase. After the anti-CRP antibodies on the immobilizing phase react with a serum sample, the resulting immobilizing phase is preferably washed with an EDTA solution. By using the antibody-PC method (B), the minimum detection limit of a sample CRP concentration can be lowered to 420 fmol. It is readily understood that PC is used after being labeled for the antibody-PC method (B).

### Effects

The novel method for determining CRP of the present invention enables accurate assay of not only serum CRP concentration during an acute phase reaction such as in the occurrence of infection, inflammation and tissue damage, but also serum CRP concentration within a normal range. The novel method for determining CRP of the present invention further allows detection of serum CRP within a normal range with a high degree of sensitivity in a rapid fashion in clinical diagnosis. In addition, because the volume of a sample required for the assay is small (100 µl per well), valuable serum samples can be saved and can thereby be subjected to the multiple assays thereby further improving accuracy.

### Examples

The materials used in the following examples are as follows.

Recombinant human C-reactive protein (rhCRP) expressed in Escherichia coli and anti-hCRP polyclonal rabbit IgG were obtained from Oriental Yeast Co., Ltd. (Japan). Phosphorylcholine (PC) chloride (calcium salt), L-α-phosphatidylcholine (L-α-lecithin derived from egg yolk), potassium hydrogen phthalate, Triton X-100 and human AB-type female sera were obtained from Sigma Chem. Co. (St. Louis, MO). Rat serum was obtained from Pel-Freeze Co. (Rogers, AR). Chelex 100 resin (sodium form) was obtained from Bio-Rad (Hercules, CA). N¹-(p-isothiocyanatebenzyl)-diethylenetriamine-N¹, N², _{N}³, N³-tetraacetic acid (DETATA-ITC) was obtained from Wallac (Gaithersburg, MD); and a 96-well microplate (cat. #21-377-204) was obtained from Fisher Scientific (Springfield, NJ). Europium nitrate, 1-(β-naphthoyl)-3,3,3-trifluoroacetone, trioctylphosphine oxide (TOPO), diethylenetriamine-N¹, N², N³-pentaacetic acid (DTPA) and its dianhydride (DTPAda) were obtained from Aldrich Chem. Co. (Milwaukee, WI). Polyvinylbenzyl lactonoylamide (PVLA) was a gift from Dr. Kazukiyo Kobayashi (Nagoya Univ., Japan). As PC conjugates of PC₇-, PC₃₅- and PC₅₈-BSA, and bovine serum albumin (BSA), those produced by the previously reported method [Stults, N., et al., (1987) Anal. Biochem. 161, 567-573] were used.

### Example 1: Comparison of assayed CRP concentrations in sera, individually using the antibody-antibody method, the PC-antibody method and the antibody-PC method

### Buffers and dissociation enhancement solution

Two types of buffers for use in CRP assay were i) TBS (20 mM Tris-HCl, pH 7.6 containing 0.15 M NaCl and 10 mM CaCl₂) and ii) TBST (TBS buffer containing 0.02% Tween 20).

The dissociation enhancement solution was composed of 15 µM β-NTA, 50 µM TOPO and 0.1% (w/v) Triton X-100 in 0.1 M acetic acid adjusted to pH 3.2 using potassium hydrogen phthalate [Hemmila, I., et al., (1984) Anal. Biochem. 137, 335-343].

### Microtiter plate coating

The anti-CRP IgG antibody coated on the plate was increased in quantity, following an increase in the IgG concentration up to 5 µg/ml. Above this level, no increase in the coated quantity was observed. For the assay on microplates and the binding assay, thus, the antibody of 500 ng was left to stand in wells for coating.

Because it was difficult to immobilize PC-BSA without modification of the microtiter plate, PC-BSA was immobilized on the wells of a microtiter plate by using the PVLA method [Suzuki, N., et al., (1997) Anal. Biochem. 247, 412-416]. PVLA (2 mg) was suspended in 1 ml of water by ultrasonication; the resulting PVLA suspension of 40 µl was added to each well, for overnight incubation at 4°C. 1 mM NaIO₄ solution was added in 50 µl-portions to each well, and the plate was incubated in darkness at ambient temperature for one hour. After the plate was washed, the PC-BSA solution (100 ng protein in 40 µl of water) was added to each well, followed by mixing with 5 µl of 2% (v/v) borane-pyridine complex, for incubation at 37°C for 2 hours. The wells were blocked through incubation with 1M glycylglycine (40 µl) at 37°C for 4 hours, and then followed by incubation with 200 µl of TBS containing 2% (w/v) BSA at 4°C overnight. After the added solutions were removed at the individual stages of the following method, the wells were washed six times with 200 µl of TBS.

To immobilize PC-BSA using the PVLA method, the level of coating was increased to 100 ng of the PC-BSA used per well, depending on the quantity of PC-BSA used. Thus, 100 ng of PC-BSA was routinely used for the PVLA method.

After preliminary tests were carried out, the level of Eu³⁺-labeled anti-CRP IgG (20 ng IgG, 1 pmol Eu³⁺/well) or PC₃₅-BSA (20 ng BSA, 1.8 pmol Eu³⁺/well) was selected, so that the background count could be minimized.

### CRP assay by DELFIA method

The principles of the three assay methods are shown in Fig. 1. Unless otherwise stated, the reagent solutions were discarded at the individual stages described, followed by washing of the wells six times with 200 µl TBST. Eu³⁺ fluorescence was finally detected by the DELFIA method previously reported [Guzaeva, T. V., et al., (1993) Immunol. Lett. 35, 285-290].

### (A) Antibody-antibody method

100 µl of TBS containing the anti-CRP antibody (500 ng) was added to each well, for overnight incubation at 4°C. Each well was blocked through incubation in 200 µl of 2% (w/v) BSA (in TBS) at ambient temperature for 2 hours; after discarding the BSA solution, the well was washed six times with 200 µl of TBS. 100 µl each of TBST samples containing variable quantities of CRP was applied to the wells coated with the antibody, followed by incubation at ambient temperature for 2 hours. After discarding the CRP solution, 100 µl of a TBST buffer solution containing 20 ng of Eu³⁺-labeled anti-CRP antibody (9 Eu³⁺ per IgG) was added to the wells. Subsequently, the plate was incubated at ambient temperature for 2 hours, to detect Eu³⁺ fluorescence.

### (B) Antibody-PC method

A microtiter plate coated with the anti-CRP antibody and blocked with BSA was prepared as described above. 100 µl each of TBST containing CRP at variable quantities were left to stand in the wells, which were incubated at ambient temperature for 2 hours. After the CRP solution was discarded from the wells, 100 µl of TBST containing Eu³⁺-labeled PC-BSA was added to the wells, for incubation at ambient temperature for 2 hours. CRP treated with TBST containing 20 mM EDTA, and BSA labeled with Eu³⁺ were added to the coated wells, which were used as a background and as a standard.

### (C) PC-antibody method

So as to immobilize PC₅₈- or PC₇-BSA solution (40 µl containing 100 µg protein and PC of about 120 pmol or 15 pmol) in water, the solution was left to stand in the PVLA-coated well. After the wells were coated with PC-BSA and blocked with BSA, they were then washed six times with TBS (200 µl). 100 µl each of TBST containing various concentrations of CRP were introduced in the wells, for incubation at ambient temperature for 2 hours. After discarding the CRP solutions, the Eu³⁺-labeled anti-CRP antibody (20 ng in 100 µl of TBST) was added, and the plate was then incubated at ambient temperature for 2 hours.

### Comparison of values of CRP concentration assayed

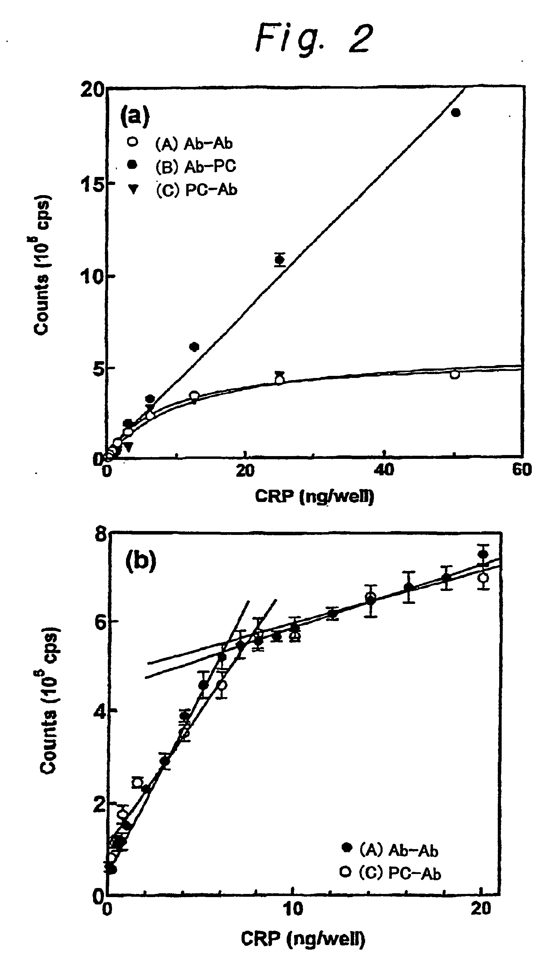

CRP assays by the antibody-antibody method (A) and the PC-antibody method (C) individually exerted two-phase standard curves (10 to 60 ng and 100 to 200 ng, respectively) (Fig. 2b). Alternatively, the results of the CRP assay by the antibody-PC method (B) were on a line within a range of 5 to 500 ng CRP/ml (Fig. 2a). The range was far wider than the ranges shown in Fig. 2b.

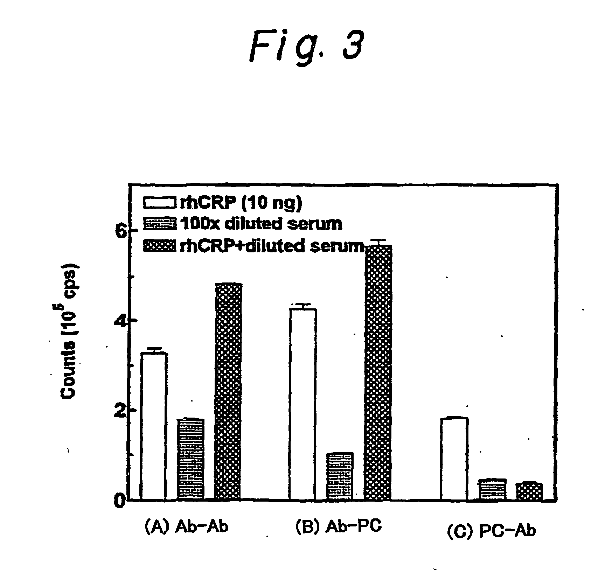

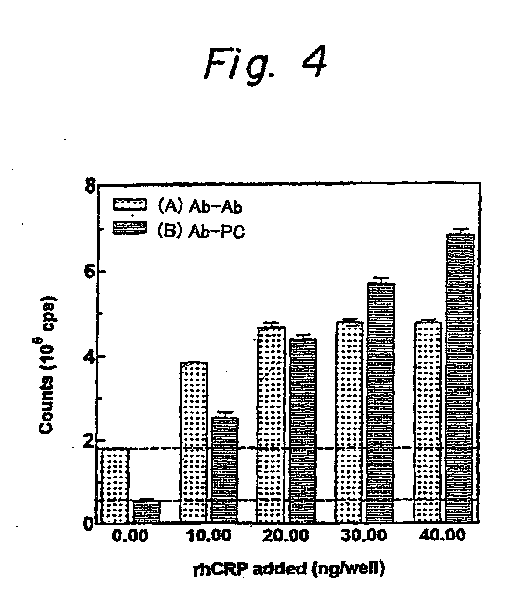

The antibody-antibody method (A) and the antibody-PC method (B) were not influenced by the presence of serum. rhCRP added to sera was not accurately determined by the PC-antibody method (C) (Fig. 3). Despite the presence or absence of BSA or PdC, rhCRP at known quantities in 100-fold diluted sera was effective in bringing about results expected from the antibody-antibody method (A) and the antibody-PC method (B). However, for the antibody-antibody method (A), the acceptable results with negligible interference were obtained only within a limited range (Fig. 4).

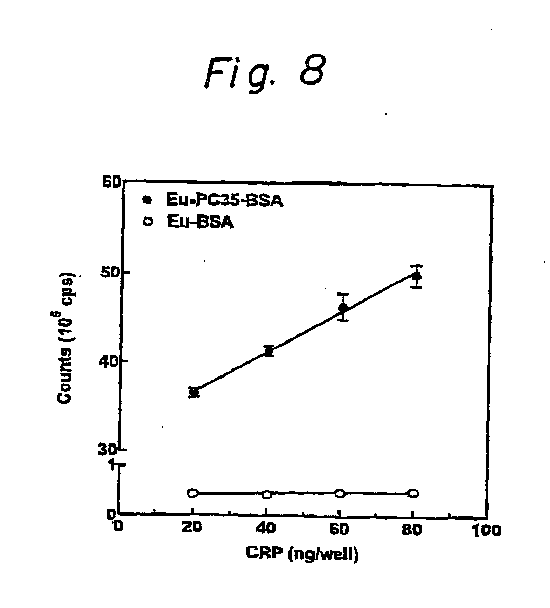

The DELFIA technique has enabled the development of a highly sensitive CRP assay method, as regards CRP assay. The degree of sensitivity and accuracy of the antibody-PC method (B) using a plate coated with the anti-CRP antibody and PC-BSA labeled with Eu³⁺ are so high that 400 fmol Eu³⁺ (namely, 420 fmol CRP) can be detected. The specific binding can be recovered by removing a non-specific binding count from the total binding count (Fig. 8). The sensitivity corresponds to the range of 5 to 500 ng CRP/ml (Fig. 2) and is comparative to that of radioimmunoassay (8 to 800 ng CRP/ml) [Claus, D. R., et al., (1976) J. Lab. Clin. Med. 87, 120-128]. The standard deviation from triplet assays was less than 3%. Further, the linear range detectable by the invented method was far wider as compared with general ELISA methods [by ELISA methods, the range was 10 to 100 ng CRP/ml (Unten, S. K., and Hokama, Y. (1987) J. Clin. Lab. Anal. 1, 136-139)]. The antibody-PC method (B) directly uses PC-BSA labeled with Eu³⁺ in place of a secondary antibody, and is thus simpler. Owing to the assay sensitivity, CRP at a normal levels in serum can also be assayed even by using a serum sample in 10- to 100-fold dilution.

### Example 2: PC-binding assays, individually using the antibody-antibody method, the PC-antibody method and the antibody-PC method.

### Buffers and dissociation enhancement solution

The two types of buffers and the dissociation enhancement solution used for PC-binding assay were prepared in the same manner as those used for CRP assay.

### Microtiter plate coating

The microtiter plate used for PC-binding assay was prepared by using anti-CRP IgG antibody or PC-BSA in the same manner as the plate used for CRP assay.

### Quantitative precipitation of CRP

Quantitative precipitation reaction was carried out as follows: variable quantities of PC₅₈-BSA in TBS was added to CRP (50 µg) in TBS to a final volume of 50 µl. After incubation at 37°C for one hour, the resulting mixture was left to stand at 4°C for 48 hours and then centrifuged (10,000 g x 5 minutes). The precipitate was washed three times with cold TBS and was then dissolved in 0.5 M NaOH (50 µl).

Protein concentration in the precipitate was determined on the basis of bicinchoninic acid (BCA) assay with a Pierce kit (Rockford, IL) by using rhCRP as a CRP standard or by using BSA as a PC-BSA standard [Smith, P. K., et al., (1985) Anal. Biochem. 193, 76-85]. When BSA is used as a standard for CRP assay, a coefficient of 0.84 should be used so as to assay accurately CRP concentration by the BCA method. Phosphoric acid was measured by using phosphorylcholine as a standard by the organic phosphoric acid analysis method of Ames, et al. [Ames, B. N., and Dubin, D. T. (1960) J. Biol. Chem. 235, 765-775].

### Interference by BSA, lecithin (PdC)and human sera

By using the aforementioned three methods, namely the antibody-antibody method (A), antibody-PC method (B) and PC-antibody method (C), CRP was assayed in a serum in 100-fold dilution with TBST. The rhCRP (10 to 40 ng/well) was added to the diluted serum, and the resulting mixture was left to stand in wells for assay. The influence of the presence of serum on these methods was compared using rhCRP (10 ng) as a standard. Similarly, TBST containing BSA (10 mg/ml) or PdC (1 mg/ml) was added to a solution containing variable quantities of rhCRP in order to examine the interference of said TBS containing TBST and PdC. As a blank test, CRP was diluted with TBST containing 20 mM EDTA.

### PC-binding assay by DELFIA method

The three assay methods used for PC-binding assay were identical to the three methods used for CRP assay. And as with CRP assay, Eu³⁺ fluorescence was finally detected according to the DELFIA method.

### PC-binding assay

CRP concentration in mol is expressed as the pentamer (molecular weight of 118,000).

### (i) Precipitation method

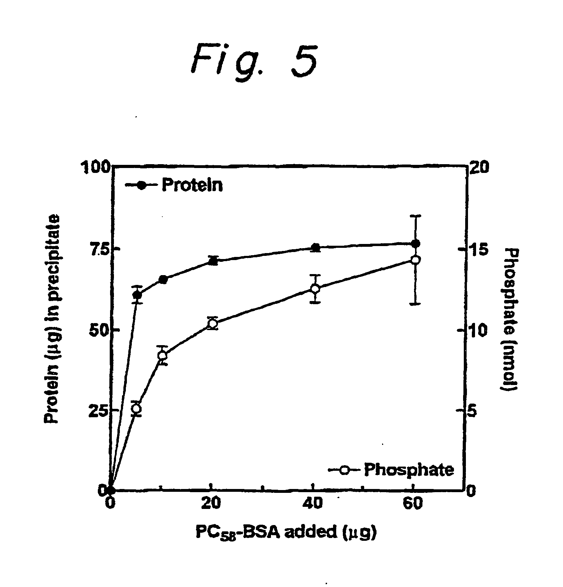

The CRP activity to bind PC was assayed through quantitative precipitation by using 10 to 80 µg PC₅₈-BSA and 50 µg CRP to determine the protein and phosphoric acid in the precipitate and assay the binding activity of CRP (Fig. 5). The precipitate reached a plateau at 20 µg of the PC-BSA level, while 10 nmol phosphoric acid was observed in 70 µg of the precipitate (as BSA).

### (ii) Microtiter plate method

Using the antibody-PC method (B) and the PC-antibody method (C), the PC-binding activity was assayed, using 20 ng of a Eu³⁺-labeled probe, within a CRP range of 5 to 100 ng per well. In the case that PC-BSA or PdC was directly coated on plastic wells, no material coated (with PC-BSA or PdC) at a sufficiently high level for the assay of the binding activity of CRP was recovered, even when a Eu³⁺-labeled probe was used (the data not shown). Thus, a PC-BSA immobilized plate was prepared by using the PVLA method.

When BSA (10 mg/ml) was present, the antibody-PC method (B) or the PC-antibody method (C) was never influenced; when 1 mM PdC (PdC concentration in serum) or diluted serum was present, the PC-antibody method (C) was influenced but the antibody-PC method (B) was not influenced (Fig. 3).

It is surmised that the interference of the serum components in the PC-antibody method (B) may be due to the reaction of PdC mainly with the PC-binding site of CRP (Fig. 3). In fact, it is known that CRP precipitates together with lecithin in the presence of calcium [Hokama, Y., et al., (1974) Clin. Chem. Acta 50, 53-62]. So as to prevent such PdC interference, therefore, it was indicated that the antibody-PC method (B) which enables anti-CRP antibodies with no relation to the PC-binding activity to capture CRP from serum samples, was preferably used. Further, after a sample containing CRP was applied to the plate, additionally, the plate was preferably washed with an EDTA solution. This is intended to remove inhibitory substances in serum from the PC-binding site (data not shown).

### Example 3: CRP activity to bind to PC

### Preparation of Ca-free PC

Phosphorylcholine chloride (calcium salt) was suspended in water, together with 100 ml of Chelex 100 (sodium type); and the resulting suspension was agitated overnight at ambient temperature. The resin was filtered under aspiration. On the filter, the resin was then washed in water. Organic phosphoric acid in the resulting aqueous PC solution (sodium salt) was analyzed and freeze-dried into powder.

### Inhibition of PC-binding activity by PC

Various amounts of PC were incubated in a total 100 µl volume of each of TBST solutions containing 2, 5, 10, 50 and 100 ng CRP, at 37°C for 2 hours. The resulting mixtures were left to stand in wells immobilized with PC₅₈-BSA, for incubation at ambient temperature for 2 hours. The CRP activity to bind to PC was assayed by using anti-CRP, as described above.

### CRP activity to bind to immobilized PC-BSA

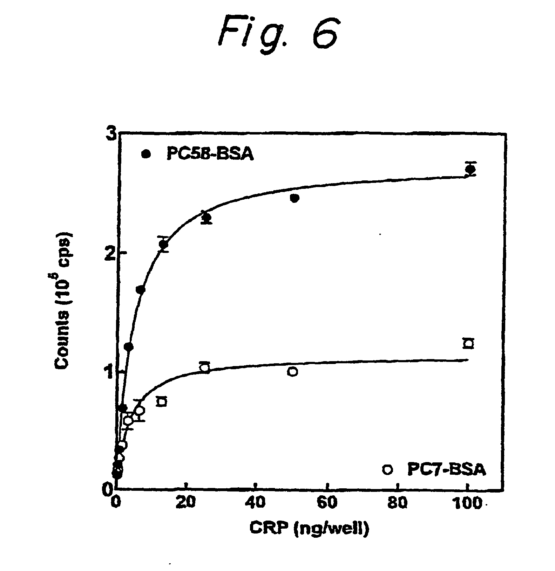

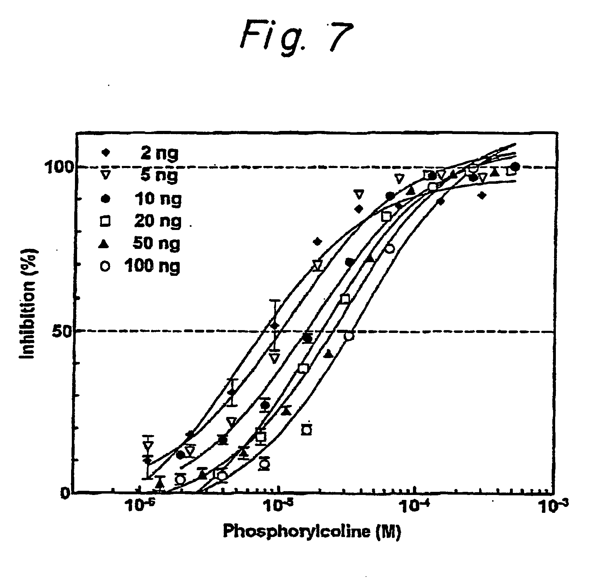

The anti-CRP antibody (IgG) was bound at 5.6 f mol and 2.2 fmol per well to PC₅₈-BSA-coated well and PC₇-BSA-coated well, respectively (Fig. 6). The inhibition potency of PC against the CRP activity to bind to the PC-BSA was assayed at various amounts of CRP. When CRP was at 2, 5, 10, 20, 50 and 100 ng, the PC concentration at 50% inhibition (IC₅₀) was 5, 11, 16, 17, 27 and 37 µM (Fig. 7) respectively, when the PC-antibody method (C) was used.

The antibody-PC method (B) has additional advantages. Most of current CRP assay methods singly depend on anti-hCRP antibody. Because human serum amyloid A (hSAA) and P(hSAP) are of the pentraxin family and with considerable homology to hCRP (66% and 70%, respectively) [Agrawal, A., et al., (1994) in Acute Phase Proteins-Molecular Biology, Biochemistry and Clinical Applications-(Machiewicz , A., et al., eds.), pp. 79-92, CRC Press, London; Shrive, A. K., et al., (1996) Nature Struc, Biol. 3, 346-354; Kilpatrick, J., and Volanakis, J. (1991) Immunol. Res. 10, 43-53], anti-hCRP antibodies possibly react with these pentraxins when the antibodies are polyclonal antibodies and used as primary antibodies for the assay. Meanwhile, only CRP is reactive with PC-BSA [Eriksen, N., et al., (1994) in Acute Phase Proteins-Molecular Biology, Biochemistry and Clinical Applications-(Machiewicz, A., et al., eds.), pp. 93-106, CRC Press, London]. According to the antibody-PC method (B), therefore, only CRP is assayed through specific reaction with PC-BSA labeled with Eu³⁺ during the final stage of the method.

At saturation precipitation when 20 µg PC₅₈-BSA was added, the precipitate contained 0.17 nmol PC₅₈-BSA (10 nmol PC in total) and 0.37 nmol CRP, and the chemical amount of PC₅₈-BSA bound to CRP was 1:2.2 (as the ratio of PC₅₈-BSA : CRP; Fig. 5). The PC-BSA immobilized by the PVLA method was approximately calculated to be 5 to 6 fmol (2.4 x 10⁵ to 2.6 x 10⁵ cps) per well based on the Eu³⁺ count according to the PC-antibody method (C), provided that the ratio of PC-BSA : CRP: anti-CRP IgG in the complex was 1 : 1: 1 (Fig. 6).

The PC-binding affinity of CRP was assayed by equilibrium dialysis at 15°C using radioactively labeled PC and capillary electrophoresis at 21°C. The results were K_{d} = 6.3 µM [Anderson, J. K., et al., (1978) Fed. Proc. 37, 1495] and K_{d} = 17.7 µM [Heegaard, N. H. H., and Robey, F. A. (1993) J. Immunol. Methods 166, 103-110]. Since PC inhibited CRP at 0.2, 0.4, 0.9, 1.7, 4.2 and 8.5 pM at 37°C by using the PC-antibody method (C), the IC₅₀ of PC was 5, 11, 16, 17, 27 and 37 µM in the order, consequently (Fig. 7). The ligand concentration at 50% inhibition is frequently used to approximately express a dissociation constant when both the receptor- and ligand concentrations are below 1% of the actual K_{d} [Chang, K.-J., et al., (1975) Biochem. Biophys. Acta 406, 294-303]. Although the amount of PC-BSA immobilized on the well is not known, the amount occupies only 0.2% of the reported K_{d} value, even if the total amount of PC-BSA is immobilized. Additionally, the CRP concentration is below 0.01% of the K_{d} value. Thus, the IC₅₀ value can approximately express binding constant (Kd). In such a case, the K_{d} value (5 to 37 µM) approximately calculated in such a manner is consistent with the results of the equilibrium dialysis and the capillary electrophoresis (6.3 to 17.7 µM).

## Claims

1. An *in vitro* method for determining a concentration of C-reactive protein (CRP) in a sample, using labelled phosphorylcholine (PC) comprising the steps of :
(i) binding an anti-CRP antibody to an immobilizing phase;
(ii) reacting a sample solution with the antibody bound to the immobilizing phase to bind the CRP in the sample to the antibody on the immobilizing phase;
(iii) reacting a labelled PC with the CRP bound to the antibody on the immobilizing phase; and
(iv) detecting the signal from the labelled PC bound on the immobilizing phase; and
(v) determining the concentration of CRP in the sample on the basis of the intensity of the signal.

2. The method for determining a concentration of CRP according to claim 1, wherein the sample containing CRP is a liquid derived from a human being.

3. The method according to claim 1 or 2, wherein PC is labelled with a radioactive labelling means or a non-radioactive labelling means.

4. The method according to claim 3, wherein the non-radioactive labelling means is a lanthanide.

5. The method for determining a concentration of CRP according to claim 4, wherein the labelling of PC with a lanthanide is carried out by labelling another substance bound to PC.

6. The method according to claim 4 or 5, wherein the lanthanide is Eu³⁺.

7. A kit for determining a concentration of CRP in a sample, comprising
(i) an immobilizing phase where an anti-CRP antibody is immobilized; and
(ii) PC labelled with a lanthanide.

8. The kit according to claim 7, wherein the lanthanide is Eu³⁺.

## Patentansprüche

1. In-vitro-Verfahren zur Bestimmung einer Konzentration von C-reaktivem Protein (CRP) in einer Probe unter Verwendung von markiertem Phosphorylcholin (PC), umfassend die folgenden Schritte:
(i) Bindung eines Anti-CRP-Antikörpers an eine immobilisierende Phase;
(ii) Umsetzung einer Probenlösung mit dem Antikörper, gebunden an die immobilisierende Phase zur Bindung des CRPs in der Probe an den Antikörper auf der immobilisierenden Phase;
(iii) Umsetzen eines markierten PC mit dem CRP, gebunden an den Antikörper auf der immobilisierenden Phase;
(iv) Nachweis des Signals von dem markierten PC, gebunden an die immobilisierende Phase und
(v) Bestimmung der CRP-Konzentration in der Probe auf der Basis der Intensität des Signals.

2. Verfahren zur Bestimmung einer Konzentration von CRP gemäß Anspruch 1, worin die Probe, die CRP enthält, eine Flüssigkeit ist, die von einem Menschen abstammt.

3. Verfahren gemäß Anspruch 1 oder 2, worin das PC mit einer radioaktiven Markierung oder einer nichtradioaktiven Markierung markiert ist.

4. Verfahren gemäß Anspruch 3, worin die nichtradioaktive Markierung ein Lanthanid bedeutet.

5. Verfahren zur Bestimmung einer Konzentration von CRP gemäß Anspruch 4, worin die Markierung von PC mit einem Lanthanid durch Markierung einer anderen Substanz, gebunden an PC, durchgeführt wird.

6. Verfahren gemäß Anspruch 4 oder 5, worin das Lanthanid Eu³⁺ ist.

7. Kit zur Bestimmung einer Konzentration von CRP in einer Probe, umfassend:
(i) eine immobilisierende Phase, an der ein Anti-CRP-Antikörper immobilisiert ist, und
(ii) PC, markiert mit einem Lanthanid.

8. Kit gemäß Anspruch 7, worin das Lanthanid Eu³⁺ ist.

## Revendications

1. Procédé *in vitro* de détermination d'une concentration en protéine réactive C (CRP) dans un échantillon, en utilisant la phosphorylcholine (PC) marquée, comprenant les étapes consistant à :
(i) lier un anticorps anti-CRP à une phase d'immobilisation ;
(ii) faire réagir une solution échantillon avec l'anticorps lié à la phase d'immobilisation pour lier la CRP dans l'échantillon à l'anticorps sur la phase d'immobilisation ;
(iii) faire réagir une PC marquée avec la CRP liée à l'anticorps sur la phase d' immobilisation ; et
(iv) détecter le signal provenant de la PC marquée liée à la phase d'immobilisation ; et
(v) déterminer la concentration en CRP dans l'échantillon sur la base de l'intensité du signal.

2. Procédé de détermination d'une concentration en CRP selon la revendication 1, où l'échantillon contenant la CRP est un liquide provenant d'un être humain.

3. Procédé selon la revendication 1 ou 2, où la PC est marquée avec un moyen de marquage radioactif ou un moyen de marquage non radioactif.

4. Procédé selon la revendication 3, où le moyen de marquage non radioactif est un lanthanide.

5. Procédé de détermination d'une concentration en CRP selon la revendication 4, dans lequel le marquage de la PC avec un lanthanide est réalisé en marquant une autre substance liée à la PC.

6. Procédé selon la revendication 4 ou 5, dans lequel le lanthanide est Eu³⁺.

7. Kit pour déterminer une concentration en CRP dans un échantillon, comprenant :
(i) une phase d'immobilisation où un anticorps anti-CRP est immobilisé ; et
(ii) une PC marquée avec un lanthanide.

8. Kit selon la revendication 7, dans lequel le lanthanide est Eu³⁺.
